# EUROPEAN PATENT SPECIFICATION

(11) **EP 3 842 689 B1**
(45) Date of publication and mention of the grant of the patent: **15.05.2024**
(21) Application number: 18925450.1
(22) Date of filing: 06.07.2018
(51) Int. Cl.: A61B 90/30, H05B 47/105, H05B 47/125, A61B 90/00, A61B 34/20, A61B 34/00

(54) **METHOD FOR ADJUSTING SURGICAL LIGHT PARAMETERS, SURGICAL LIGHTING DEVICE, AND READABLE STORAGE MEDIUM**
VERFAHREN ZUR EINSTELLUNG DER PARAMETER EINER OP-LEUCHTE, OP-LEUCHTE UND LESBARES SPEICHERMEDIUM
PROCÉDÉ DE RÉGLAGE DE PARAMÈTRES D'ÉCLAIRAGE CHIRURGICAL, DISPOSITIF D'ÉCLAIRAGE CHIRUGICAL ET SUPPORT D'INFORMATIONS LISIBLE

(43) Date of publication of application: 30.06.2021
(73) Proprietor: Nanjing Mindray Bio-Medical Electronics Co., Ltd., Nanjing, Jiangsu 211111 (CN)
(72) Inventor: CHEN, Xiaokai, Nanjing, Jiangsu 211111 (CN); WANG, Dan, Nanjing, Jiangsu 211111 (CN)
(74) Representative: KIPA AB
(86) International application number: PCT/CN2018/094911
(87) International publication number: WO 2020/006766

(56) References cited:
- EP-A1- 3 815 471
- CN-A- 101 858 537
- CN-A- 104 320 881
- CN-A- 104 363 676
- CN-A- 106 500 017
- CN-A- 109 073 176
- DE-A1-102013 014 078
- DE-A1-102014 214 881
- US-A1- 2009 273 287
- US-A1- 2011 037 840
- US-A1- 2017 318 644

## Description

### Technical Field

This disclosure relates to the field of medical equipment, and in particular to a method for adjusting parameter(s) of a surgical light, a surgical lighting device, and a readable storage medium.

### Background Art

Clinically, surgeons may perform surgeries on different biological tissue sites, and different surgical sites correspond to different surgery types. Different surgery types such as an abdominal surgery, a cardiac surgery, and a spine surgery have different requirements on the light spot size and illuminance of a surgical light. In addition, requirements on surgical light parameters are also different at different surgery stages. For example, in an early surgery period, the surgeons mainly perform operations such as disinfecting a surgical site and making an incision. During a surgery, the surgeons often need to perform refinement operations on the surgical site, such as a refinement operation inside a cavity. In a late surgery period, the surgeons need to perform a surgical suture operation. For different operation objects at different stages, required parameters such as the light spot size, the illuminance, and the angle of the surgical light may be different.

EP 3815471 A1 is prior art under Art. 54(3) EPC and describes a lighting system and associated controls that can provice active control of lighting device settings.

US 2017/0318644 A1 describes a surgical lamp for illuminating a surgical field on a human body. The surgical lamp comprises a control device, a lamp body comprising several illuminants with one respective light ray directed to the surgical field, and a 3D sensor for detecting a spatial position of at least one object. The control device is configured to control the illuminants according to the spatial position of the at least one object.

US 2009/0273287 A1 describes a lighting system for delivering a dynamic, fully customized, and automatic illumination to a subject. The lighting system comprises a programmable light unit for emitting a programmed pattern and spectra of illumination. The lighting system generates light in accordance with the lighting profile, which is fully optimized in spectrum, intensity, color, contrast, temperature, angle, and focus for any given environment, subject and task.

DE 10 2013 014 078 A1 describes a lighting system where the operating area should be optimally and automatically illuminated by means of 3D localization and automatic alignment of the operating room lights. Alignment is calculated using 3D localization.

### Summary of the Invention

This disclosure mainly provides a method for adjusting parameter(s) of a surgical light, a surgical lighting device, and a readable storage medium, and is aimed to automatically adjust surgical light parameter(s) according to an acquired operation field image signal or an acquired three-dimensional signal of an operation field.

The invention is defined by the appended independent claims.

According to the method for adjusting the parameter(s) of the surgical light, the surgical lighting device, and the computer-readable storage medium in the embodiments described above, the operation field image signal or the three-dimensional signal of the operation field is acquired, and surgery information is determined according to the operation field image signal or the three-dimensional signal of the operation field, the surgery information comprising the surgery type or the surgery stage; then the parameter adjustment strategy for the surgical light is determined according to the surgery information, and surgical light parameters are adjusted according to the parameter adjustment strategy. It can be seen that the parameter(s) of the surgical light may be automatically adjusted by means of acquiring the operation field image signal or the three-dimensional signal of the operation field in the present invention, thus freeing both hands of medical personnel.

### Brief Description of the Drawings

Fig. 1 is a structural block diagram of a surgical lighting device provided by the present invention;
Fig. 2 is a structural block diagram of a processor in a surgical lighting device provided by the present invention;
Fig. 3 is a schematic diagram of a surgery region in an operation field image in an early surgery period in a system for adjusting parameter(s) of a surgical light provided by the present invention;
Fig. 4 is a schematic diagram of a surgery region when an incision is being made during a surgery in a system for adjusting parameter(s) of a surgical light provided by the present invention;
Fig. 5 is a schematic diagram of a surgery region after an incision is made during a surgery in a system for adjusting parameter(s) of a surgical light provided by the present invention;
Fig. 6 is a schematic diagram of a surgery region in a late surgery period in a system for adjusting parameter(s) of a surgical light provided by the present invention; and
Fig. 7 is a flowchart of a method for adjusting parameter(s) of a surgical light provided by the present invention.

### Detailed Description of Embodiments

The present invention will be further described in detail below through specific embodiments in conjunction with the accompanying drawings. Associated similar element reference numerals are used for similar elements in various embodiments. In the following embodiments, many details are described such that the present application can be better understood. However, it would have been effortlessly appreciated by those skilled in the art that some of the features could be omitted or could be substituted by other elements, materials, and methods in different cases. In certain cases, some operations involved in the present application are not displayed or described in the specification, which is to prevent the core part of the present application from being obscured by too much description. Moreover, for those skilled in the art, the detailed description of the involved operations is not necessary, and the involved operations can be thoroughly understood according to the description in the specification and the general technical knowledge in the art.

In addition, the characteristics, operations, or features described in the specification can be combined in any appropriate manner to form various embodiments. Moreover, the steps or actions in the method description can also be exchanged or adjusted in order in a way that would have been obvious to those skilled in the art. Therefore, the various orders in the specification and the accompanying drawings are merely for the purpose of clear description of a certain embodiment and are not meant to be a necessary order unless otherwise stated that a certain order must be followed.

The serial numbers themselves for the components herein, for example, "first", "second", etc., are merely used to distinguish the described objects, and do not have any sequential or technical meaning. Moreover, as used in the present application, "connection" or "coupling", unless otherwise specified, comprises both direct and indirect connections (couplings).

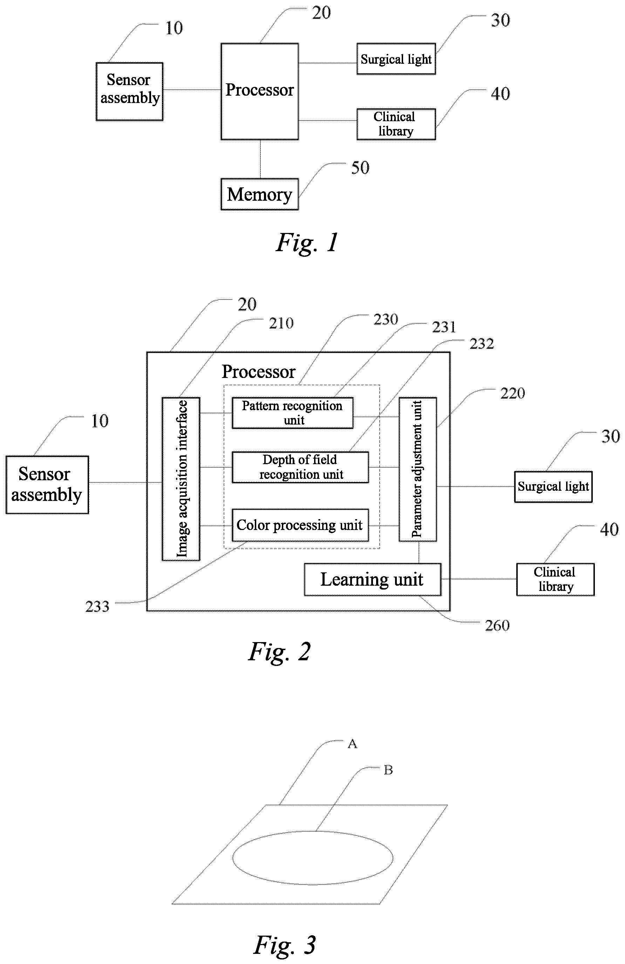

As shown in Fig. 1 and Fig. 2, a surgical lighting device provided by the present invention can include a surgical light 30 and a system for adjusting parameter(s) of the surgical light. The surgical light 30 is configured to project a light spot on a surgical site according to predetermined parameters. The system for adjusting the parameter(s) of the surgical light is configured to adjust the parameter(s) of the surgical light. The system for adjusting the parameter(s) of the surgical light includes a processor 20. The processor 20 includes an image acquisition interface 210, a processing unit 230, and a parameter adjustment unit 220. The image acquisition interface 210 is connected to the parameter adjustment unit 220 through the processing unit 230.

The image acquisition interface 210 is configured to receive an operation field image signal or a three-dimensional signal of an operation field. The operation field image signal is an electrical signal of an operation field image that is transmitted from the outside of the processor. For example, the image acquisition interface 210 can receive a real-time operation field image signal output by a camera or operation field image signals output by other electronic devices. The three-dimensional signal of the operation field is an electrical signal containing a three-dimensional feature of the operation field that is transmitted from the outside of the processor. For example, the image acquisition interface 210 may receive a real-time three-dimensional signal of the operation field that is output by a 3D image sensor or three-dimensional signals output by other electronic devices.

The processing unit 230 is configured to determine surgery information according to the operation field image signal or the three-dimensional signal of the operation field, where the surgery information includes a surgery type or a surgery stage. In other words, the surgery information may include one of the surgery type and the surgery stage, or may include both the surgery type and the surgery stage. In this embodiment, an example in which the surgery information includes both the surgery type and the surgery stage is used for description. Similarly, the surgery information may be determined according to the operation field image signal, or the surgery information may be determined according to the three-dimensional signal of the operation field, or the surgery information may be determined according to both the operation field image signal and the three-dimensional signal of the operation field.

The parameter adjustment unit 220 is configured to determine a parameter adjustment strategy for the surgical light according to the surgery information, and adjust the parameter(s) of the surgical light according to the parameter adjustment strategy. The parameter(s) of the surgical light include but is/are not limited to the illuminance, the light spot size, the color temperature, the irradiation angle, and the like of the surgical light. In the present invention, the operation field image signal or the three-dimensional signal of the operation field is acquired, a current surgery type or a current surgery stage is determined according to the operation field image signal or the three-dimensional signal of the operation field, and then the parameter adjustment strategy for the surgical light is determined, so that the parameter(s) of the surgical light can be automatically adjusted, thus freeing both hands of medical personnel.

The system for adjusting the parameter(s) of the surgical light further includes a sensor assembly 10, which is configured to generate the operation field image signal or the three-dimensional signal of the operation field. For example, the sensor assembly 10 includes a camera assembly, which is configured to capture an image or a video of the operation field region and output the same to the processor 20. The camera assembly 10 may be a camera, a video camera, or the like. In this embodiment, the camera assembly 10 includes an optical lens and an image sensor. The optical lens is configured to face a scene within a range of the operation field. The image sensor is configured to image the scene within the range of the operation field on a target surface of the image sensor and output a real-time electrical signal reflecting content of the operation field image. The image sensor is electrically connected to the image acquisition interface 210. The image acquisition interface 210 acquires, in real time, the operation field image signal output by the image sensor. In other embodiments, the sensor assembly 10 may include a 3D image sensor, which uses monocular vision, binocular stereo vision, multi-view stereo vision, a structured light method, a time-of-flight (ToF) method, a triangulation method, or the like to acquire the three-dimensional signal of the operation field in real time. For example, a 3D distance sensor or a binocular (camera) is used to perform distance measurement. The three-dimensional signal of the operation field can be synthesized into a three-dimensional image of the operation field, and the surgery information may be determined after the three-dimensional signal is modeled to obtain the three-dimensional image, or predetermined feature information in the operation field may be obtained from the three-dimensional signal.

Specifically, when the processing unit 230 determines the surgery information according to the operation field image signal or the three-dimensional signal of the operation field, the processing unit 230 may obtain predetermined feature information in the operation field image or the three-dimensional signal according to the operation field image signal or the three-dimensional signal of the operation field; and determine, according to the predetermined feature information, the surgery information corresponding to the feature information. That is, the predetermined feature information, such as predetermined depth information of an incision, color information, an incision size, human body posture information, surgery bed placement position information, brightness information, and human body information, can be searched in the operation field image or the three-dimensional signal acquired in real time. Specifically, the three-dimensional signal may be searched for feature information with three-dimensional features such as depth information of an incision, an incision size, human body posture information, surgery bed placement position information, and human body information. The human body information includes at least one of human body structure information and dynamic organ feature information. The processing unit 230 performs processing and determination on the feature information, and a current surgery type and a current surgery stage can be determined according to a determination result.

Medical personnel need to set different parameters for the surgical light for different surgery types, and different parameters of the surgical light also need to be set at different surgery stages for a same surgery type. The system for adjusting the parameter(s) of the surgical light in this disclosure may automatically recognize the current surgery type, adjust the parameter(s) of the surgical light, then recognize a surgery stage, and further adjust the parameter(s) of the surgical light based on the surgery type. A specific process is shown in the following embodiment.

The processor 20 further includes a storage unit (not shown in the figure) and a learning unit 260. The processing unit 230 includes a pattern recognition unit 231, a depth of field recognition unit 232, and a color processing unit 233.

The storage unit stores the human body posture information corresponding to the surgery bed placement position information. In other words, the storage unit stores a correspondence (such as a correspondence table) between the surgery bed placement position information and the human body posture information, and may further include a correspondence between the human body posture information and the surgery type.

The pattern recognition unit 231 is configured to: obtain the human body posture information and the surgery bed placement position information in the operation field image or the three-dimensional signal according to the operation field image signal or the three-dimensional signal of the operation field. For example, predetermined human body posture information and surgery bed placement position information can be searched in the operation field image or the three-dimensional signal acquired in real time; the surgery type corresponding to the human body posture information and the surgery bed placement position information can then be determined according to the human body posture information and the surgery bed placement position information. For example, preset human body posture information corresponding to the surgery bed placement position information may be searched according to current surgery bed placement position information; and it is then determined whether a current human body posture information matches the preset human body posture information obtained by searching. Different surgery types may require patients to present different postures such as a sitting posture and a lying posture, and different surgery types may correspond to different surgery bed placement positions. When the current human body posture information matches the preset human body posture information obtained by searching, it is considered that the patient is already on the surgery bed and is waiting for a surgery. The pattern recognition unit 231 preliminarily recognizes a surgical site according to a current human body posture, and then obtains a current surgery type.

The surgical lights in different states are required for different surgical sites. For example, a spinal surgery requires a surgical light with a small light spot and high illuminance. The parameter adjustment unit 220 determines the parameter adjustment strategy for the surgical light according to the current surgery type, and adjusts the parameter(s) of the surgical light accordingly. For example, if the surgery type is a cardiac surgery, the surgical light is adjusted to a mode for the cardiac surgery.

The number of the surgery type that can be determined according to the human body posture information is not large, and thus the pattern recognition unit 231 further has a function of recognizing a surgical site. For example, the pattern recognition unit 231 obtains the brightness information and the human body information in the operation field image according to the operation field image signal; and determines, according to the brightness information and the human body information, the surgery type corresponding to the brightness information and the human body information. Specifically, the pattern recognition unit 231 searches for predetermined brightness information, human body structure information, and dynamic organ feature information, in the operation field image acquired in real time; and connects pixel points whose differences in brightness values do not exceed a preset value in the brightness information of the current operation field image to form one region, and compares average brightness values of all regions obtained after the connection, to obtain a region with the largest average brightness value. The region with the largest average brightness value is considered as the light spot formed by the surgical light irradiating the patient, and a surgical site is certainly within the light spot (the surgical light can be directed to the surgical site by medical personnel or by the parameter adjustment unit 220). Therefore, the current surgical site is obtained according to the human body structure information and/or the dynamic organ feature information in the region with the largest average brightness value, thereby obtaining the current surgery type. The preset value may be determined according to an actual situation of the surgical light. The more uniform the light spot of the surgical light, the smaller the preset value. The human body structure information is information reflecting features of a human body structure, such as human body site information and tissue organ information. The human body site information is information reflecting features of an external structure of the human body, for example, a head, an abdomen, a back, and a leg. The tissue organ information is information reflecting structural features of an internal tissue and organ of the human body, for example, a heart, intestines, a lung, a liver, a spleen, a kidney and other organs, as well as a spine. The surgical site is determined according to the human body structure information in the light spot, thereby further improving the accuracy of determining the surgery type. Certainly, a same surgical site may also correspond to different surgery types. For example, an eye surgery may be a surgery on an eyeball or a surgery on a lower eyelid. Therefore, determining the surgery type according to the dynamic organ feature information in the light spot can further improve the accuracy of determining the surgery type. The dynamic organ feature information includes blinking, heart beating, lung expansion and contraction, gastrointestinal peristalsis, and the like, which can provide a basis for determining the surgery type.

Certainly, the surgery type may be further determined according to medical device information and/or medical instrument information in the operation field image. That is, the feature information includes the medical device information and/or the medical instrument information, for example, a ventilator, a scalpel, surgical scissors, and a draw hook.

In the present invention, the sensor assembly 10 collects the operation field image or the three-dimensional signal of the operation field in real time, and transmits the same in a form of an electrical signal (the operation field image signal or the three-dimensional signal). The operation field image in an early surgery period is a panoramic large-scale image. The human body structure information such as a head and a foot of the human body may be recognized based on the image, and a target region of a surgery is preliminarily determined based on the surgery bed placement position and a site irradiated by the light spot of the surgical light, so as to adjust the parameter(s) of the surgical light according to the determined surgical site, thereby implementing automatic coarse adjustment on the parameter(s) of the surgical light.

After the parameter(s) of the surgical light is/are adjusted according to the surgery type, a surgery stage for the surgery type may be further determined.

In this embodiment, a depth of field recognition unit 232 is configured to: obtain a current depth information of an incision in the operation field image or the three-dimensional signal according to the operation field image signal or the three-dimensional signal of the operation field. For example, predetermined depth information of an incision may be searched in the operation field image or the three-dimensional signal acquired in real time, a change value of the current depth information of the incision is then calculated in real time; the surgery stage of the current surgery is determined according to the change value of the current depth information or a change trend of the current depth information, where the surgery stage includes the early surgery period, the middle surgery period, and the late surgery period. For example, when the depth of field recognition unit 232 determines the corresponding surgery stage according to the change value of the current depth information:, it is considered that the current surgery is in the early surgery period when an absolute value of the change value of the depth information is less than or equal to a first predetermined value from the beginning of the surgery to the present; when it is continuously detected that the change value of the depth information is greater than the first predetermined value from the beginning of the surgery to the present, it is considered that the current surgery is in the middle surgery period; or when the change value of the depth information is a negative value and the absolute value thereof is greater than a second predetermined value, it is considered that the current surgery is in the late surgery period. As shown in Fig. 3 to Fig. 6, A is a surgical region enclosed by a sterile drape, B is a light spot, and C is an incision. When determining the surgery stage, the processing unit 230 only needs to process the image in the surgical region in the operation field image, thereby reducing a data processing amount and increasing a response speed. The processing unit 230 searches, for predetermined color information, the operation field image acquired in real time; determines whether there is a sterile drape color (such as light green) in color information of the current operation field image; connects pixel points with the sterile drape color to form one region, the region being usually an annular region; and determines whether there is a human skin color within the annular region. If there is a human skin color within the annular region, a part without the sterile drape color in the annular region is the surgical region.

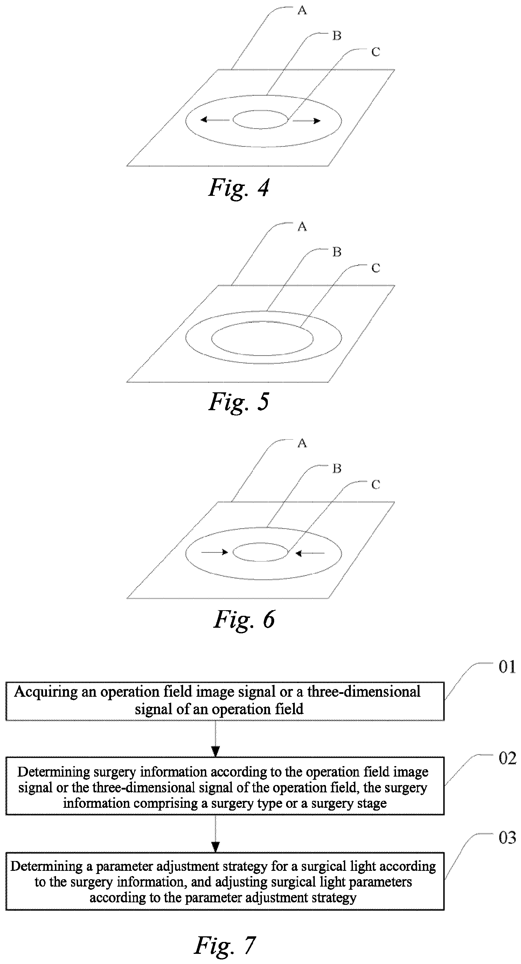

Fig. 3 is a schematic diagram of a surgical region in an operation field image in an early surgery period, namely, a disinfection stage. In this case, there is no incision, or the incision is very small, and the change value of the depth information is less than or equal to the first predetermined value. Fig. 4 is a schematic diagram of a surgical region during an incision operation in a middle surgery period. The change value of the depth information is increasing. Fig. 5 is a schematic diagram of a surgical region in an operation field image when a refinement operation is performed after an incision is made in a middle surgery period. In this case, the depth information is basically unchanged, and the change value of the depth information from the beginning of the surgery to the present is the largest. Fig. 6 is a schematic diagram of a surgical region in an operation field image when a suture operation is being performed in a late surgery period. The change value of the depth information is a negative value, and the absolute value of the change value of the depth information is greater than the second predetermined value. The depth of the incision undergoes a process of being basically 0, increasing, basically keeping unchanged, and decreasing from the early surgery period, the middle surgery period to the late surgery period, and thus the surgery stage determined according to the change value of the depth information is accurate and reliable. The first predetermined value and the second predetermined value may be determined according to the surgery type.

For example, the depth of field recognition unit 232 determines the surgery stage of the current surgery according to the change trend of the current depth information. In this example, the depth of field recognition unit 232 consecutively compares the current depth information with previous depth information for n times in a predetermined time period. When the current depth information is consecutively greater than the previous depth information for n times, the change trend of the current depth information is an increasing trend, and the current surgery is considered to be in the middle surgery period. When the current depth information is consecutively less than the previous depth information for n times, the change trend of the current depth information is a decreasing trend, and the current surgery is considered to be in the late surgery period. When the current depth information is consecutively the same as the previous depth information for n times, and the current depth information is basically 0, the current surgery is considered to be in the early surgery period. Or, when the current depth information is consecutively the same as the previous depth information for n times, and the current depth information is greater than or equal to a third predetermined value, the current surgery is considered to be in the middle surgery period. Herein, n is greater than or equal to 3, and the predetermined time period and the third predetermined value may be set according to actual requirements.

The depth of field recognition unit 232 may further establish a three-dimensional model of the operation field according to the three-dimensional signal of the operation field; determine an incision size according to the three-dimensional model, and determine the surgery stage of thecurrent surgery according to the incision size. The incision size is determined according to the three-dimensional model. For example, the depth information of each point in the three-dimensional model is obtained, a difference between the current depth information of each point and the depth information of each point before the surgery is calculated, and the points whose differences are greater than a fourth predetermined value are connected to form one region, where the region is the incision region and the incision size is then determined. Because the depth of the incision is greater than that of its surrounding region, the incision can be recognized from the three-dimensional model according to the depth information, so as to obtain the accurate incision size. The fourth predetermined value may be set according to an actual situation.

After the depth of field recognition unit 232 recognizes the surgery stage of the current surgery, the parameter adjustment unit 220 adjusts the illuminance of the surgical light to predetermined first illuminance when the current surgery is in the early surgery period.

The parameter adjustment unit 220 adjusts the illuminance of the surgical light to second illuminance when the current surgery is in the middle surgery period, and sets the second illuminance to vary according to the change in the depth information of the incision. For example, the depth information of the incision is obtained in real time, and the parameter(s) of the surgical light is/are adjusted according to the depth information of the incision, so that at least the illuminance of the surgical light increases with an increase in the depth, and medical personnel does not need to set the illuminance manually.

The parameter adjustment unit 220 adjusts the illuminance of the surgical light to third illuminance when the current surgery is in the late surgery period, and sets the third illuminance to vary depending on a change in the depth information of the incision. For example, the depth information of the incision is obtained in real time, and the parameter(s) of the surgical light are adjusted according to the depth information of the incision, so that at least the illuminance of the surgical light decreases with a decrease in the depth. Both the first illuminance and the third illuminance are less than or equal to the second illuminance. The first illuminance and the third illuminance are in a low illuminance range relative to an illuminance range of the surgical light. The low illuminance range is a relative concept, for example, the illuminance range of the surgical light may be classified into a high illuminance range and a low illuminance range, or a high illuminance range, a medium illuminance range, and a low illuminance range. The second illuminance may be in the high illuminance range or the medium illuminance range.

In this embodiment, a color processing unit 233 is configured to: obtain color information in the operation field image according to the operation field image signal. For example, predetermined color information can be searched in the operation field image acquired in real time; and determine the surgery stage of the current surgery according to the color information. For example, an area of a blood stain region in the operation field image can be determined according to the color information, an incision size may then be determined according to the area of the blood stain region, and the surgery stage of the current surgery can be determined according to the incision size. Specifically, the color processing unit 233 determines whether there is red in the color information of the current operation field image; when there is no red in the color information, the color processing unit determines that the current surgery is in the early surgery period; or when there is red in the color information, the color processing unit determines that the current surgery is in the middle surgery period or the late surgery period. Red is used to determine whether there is an incision to further determine the surgery stage, which is accurate and reliable. Further, the color processing unit 233 is further configured to connect red pixel points in the color information of the current operation field image to form one region, where the region is the blood stain region. Then, the area of the blood stain region is calculated and the incision size is determined accordingly based on the calculated area. After the incision size and the surgery stage are obtained, parameters such as the light spot size and illuminance of the surgical light may be accurately adjusted. In other embodiments, the depth of field recognition unit 232 is replaced by the color processing unit 233.

The parameter adjustment unit 220 is further configured to adjust the light spot size of the surgical light to a predetermined first light spot value when the current surgery is in the early surgery period. A light spot value reflects a size of a light spot formed by the surgical light irradiating a human body or a surgery bed. The light spot value in this embodiment is a size of a light spot formed by the surgical light irradiating the surgery bed at a distance of one meter, and is represented by a diameter.

The light spot size of the surgical light is adjusted to a second light spot value and the second light spot value varies depending on the change in the incision size when the current surgery is in the middle surgery period. For example, the incision size is obtained in real time, and the parameter(s) of the surgical light is/are adjusted according to the incision size, so that at least the light spot size or the illuminance of the surgical light increases with an increase in the incision size.

The light spot size of the surgical light is adjusted to a third light spot value and the third light spot value varies according to a change in the incision size when the current surgery is in the late surgery period. For example, the incision size is obtained in real time, and the parameter(s) of the surgical light is/are adjusted according to the incision size, so that at least the light spot size or the illuminance of the surgical light decreases with a decrease in the incision size.

Both the second light spot value and the third light spot value are less than or equal to the first light spot value, and the first light spot value is in a large light spot range relative to a light spot range of the surgical light. The large light spot range is also a relative concept. For example, the light spot range of the surgical light may be classified into a large light spot range and a small light spot range, or a large light spot range, a medium light spot range, and a small light spot range. The second light spot value and the third light spot value are in the small light spot range or the medium light spot range.

For example, as shown in Fig. 3, when the surgery is in the early surgery period, namely, a disinfection stage, the depth information is very small, and the parameter adjustment unit 220 controls the illuminance of the surgical light to be low and the light spot to be a large light spot. As shown in Fig. 4, when the surgery is in an incision stage in the middle surgery period, the incision gradually becomes larger and deeper, and the parameter adjustment unit 220 controls the light spot size and the illuminance of the surgical light to increase as the incision size increases and the depth increases. As shown in Fig. 5, when the surgery is in a refinement operation stage in the middle surgery period, the incision size and the depth basically keep unchanged, and the parameter adjustment unit 220 controls the light spot size of the surgical light to be basically the same as a size of a surgery opening to reduce the damage to human eyes caused by reflection of surgical instrument, and the parameter adjustment unit 220 controls the illuminance to be appropriate according to the depth information. As shown in Fig. 6, when the surgery is in the late surgery period, namely, a suture stage, the parameter adjustment unit 220 controls the illuminance and the light spot of the surgical light to decrease as the incision size decreases and the depth decreases. In this way, the parameter(s) of the surgical light is/are automatically adjusted with a change in the surgery stage.

The surgery type and the surgery stage may be determined simultaneously or may be determined successively or in real time. However, a priority of determining the parameter adjustment strategy for the surgical light according to the surgery type is higher than that of determining the parameter adjustment strategy for the surgical light according to the surgery stage.

The system for adjusting the parameter(s) of the surgical light provided by the present invention further includes a clinical library 40, and the processor 20 further includes a learning unit 260. The clinical library 40 is configured to store parameter adjustment strategies for the surgical lights, where the parameter adjustment strategies correspond to a plurality of surgery information. Specifically, the clinical library stores a parameter group of the surgical light and a parameter adjustment process corresponding to each surgery type, and the clinical library also stores a parameter group of the surgical light and a corresponding parameter adjustment process corresponding to each surgery stage for a same surgery type. Due to different habits and preferences of various surgeons, the parameter adjustment strategies stored in the clinical library 40 may not be the most appropriate. Therefore, the learning unit 260 is provided to record the parameter adjustment strategy of the surgical light during an actual surgery, that is, to record actual parameters of the surgical light in the current surgery type or surgery stage. The parameter adjustment strategy for the surgical light in the clinical library 40 is dynamically adjusted through deep learning, and a correspondence between a surgeon and a parameter adjustment strategy for a surgical light is established, so that the parameter(s) of the surgical light can not only be automatically adjusted according to the surgery type and the surgery stage, but also the parameter(s) can be slightly adjusted for different surgeons. In this way, a number of times the surgeon adjusts the surgical light is reduced and the surgeon's eyesight is protected during the surgery.

Based on the system for adjusting the parameter(s) of the surgical light provided by the foregoing embodiment, Fig. 7 shows a method for adjusting parameter(s) of a surgical light provided by the present invention, where the method includes the following steps.

At step 01, an image acquisition interface acquires an operation field image signal or a three-dimensional signal of an operation field. Specifically, the operation field image signal or the three-dimensional signal of the operation field that are output by a sensor assembly are acquired in real time.

At step 02, a processing unit determines surgery information according to the operation field image signal or the three-dimensional signal of the operation field, where the surgery information includes a surgery type or a surgery stage. Specifically, the processing unit obtains feature information in an operation field image or the three-dimensional signal according to the operation field image signal or the three-dimensional signal of the operation field. The processing unit then determines, according to the feature information, the surgery information corresponding to the feature information. For example, the processing unit may search for the predetermined feature information in the operation field image or the three-dimensional signal acquired in real time. Then, the processing unit performs processing and determination on the feature information to obtain the current surgery information according to a determination result.

The feature information includes human body posture information and surgery bed placement position information, and the surgery information is a surgery type. Step 02 specifically includes the following embodiments.

A pattern recognition unit obtains current human body posture information and current surgery bed placement position information in the operation field image or the three-dimensional signal according to the operation field image signal or the three-dimensional signal of the operation field. The pattern recognition unit determines, according to the current human body posture information and the current surgery bed placement position information, the surgery type corresponding to the current human body posture information and the current surgery bed placement position information. For example, the pattern recognition unit searches for, according to current surgery bed placement position information, predetermined human body posture information corresponding to the surgery bed placement position information. The pattern recognition unit determines whether the current human body posture information matches the predetermined human body posture information obtained by searching; and obtains the current surgery type according to the current human body posture information if the current human body posture information matches the predetermined human body posture information obtained by searching.

The feature information may further include: brightness information, and human body structure information and/or dynamic organ feature information, and the surgery information is a surgery type. Step 02 specifically includes the following embodiment.

A pattern recognition unit obtains brightness information and human body information in the operation field image according to the operation field image signal, where the human body information includes human body structure information or dynamic organ feature information. The pattern recognition unit determines, according to the brightness information and the human body information, a surgery type corresponding to the brightness information and the human body information. For example, the pattern recognition unit, according to the brightness information of the current operation field image, connects pixel points whose difference in brightness values do not exceed a preset value to form one region; compares average brightness values of all regions obtained after the connection to obtain a region with the largest average brightness value; and obtains the current surgery type according to the human body structure information or the dynamic organ feature information in the region with the largest average brightness value.

The feature information may further include the depth information of an incision, and the surgery information is a surgery stage. Step 02 specifically includes the following embodiment.

A depth of field recognition unit obtains depth information of an incision in the operation field image or the three-dimensional signal according to the operation field image signal or the three-dimensional signal of the operation field, calculates a change value of the depth information of the incision in real time; and determines, according to the change value of the depth information of the incision or a change trend of the current depth information, the surgery stage corresponding to the change value of the depth information of the incision or the change trend of the current depth information, where the surgery stage includes an early surgery period, a middle surgery period, and a late surgery period.

The feature information may further include color information and an incision size, and the surgery information is a surgery stage. Step 02 specifically includes the following embodiment.

A color processing unit obtains color information in the operation field image according to the operation field image signal; and determines a surgery stage of a current surgery according to the color information. For example, an area of a blood stain region in the operation field image is determined according to the color information, an incision size is determined according to the area of the blood stain region, and the surgery stage of the current surgery is determined according to the incision size. Specifically, the color processing unit determines whether there is red in the color information of the current operation field image; determines that the current surgery is in the early surgery period if there is no red in the color information; or determines that the current surgery is in a middle surgery period or a late surgery period if there is red in the color information. The color processing unit connects red pixel points in the color information of the current operation field image to form one region, where this region is the blood stain region and the area of the blood stain region is calculated to determine the incision size accordingly.

At step 03, a processor determines a parameter adjustment strategy for a surgical light according to the surgery information. The following steps are specifically included.

When the current surgery is in the early surgery period, a parameter adjustment unit adjusts the illuminance of the surgical light to predetermined first illuminance, and adjusts the light spot size of the surgical light to a predetermined first light spot value. A light spot value reflects a size of a light spot formed by the surgical light irradiating a human body or a surgery bed. The light spot value in this embodiment is a size of a light spot formed by the surgical light irradiating the surgery bed at a distance of one meter, and is represented by a diameter.

The parameter adjustment unit adjusts the illuminance of the surgical light to second illuminance, and sets the second illuminance to vary depending on a change in the depth information of the incision when the current surgery is in the middle surgery period. The parameter adjustment unit obtains the depth information of the incision in real time, and adjusts the parameter(s) of the surgical light according to the depth information of the incision, so that at least the illuminance of the surgical light increases with an increase in the depth. The parameter adjustment unit adjusts the light spot size of the surgical light to a second light spot value, where the second light spot value varies depending on a change in the incision size. For example, the parameter adjustment unit obtains the incision size in real time, and adjusts the parameter(s) of the surgical light according to the incision size, so that at least the light spot size of the surgical light increases with an increase in the incision size.

The illuminance of the surgical light is adjusted to third illuminance and the third illuminance varies depending on a change in the depth information of the incision when a current surgery is in the late surgery period. For example, the parameter adjustment unit obtains the depth information of the incision in real time, and adjusts the parameter(s) of the surgical light according to the depth information of the incision, so that at least the illuminance of the surgical light decreases with a decrease in a depth. The parameter adjustment unit also adjusts the light spot size of the surgical light to a third light spot value, and the third light spot value varies depending on a change in the incision size. For example, the incision size is obtained in real time, and the parameter(s) of the surgical light is/are adjusted according to the incision size, so that at least the light spot size of the surgical light decreases with a decrease in the incision size. Both the second light spot value and the third light spot value are less than or equal to the first light spot value, and the first light spot value is in a large light spot range relative to a light spot range of the surgical light. The large light spot range is also a relative concept. For example, the light spot range of the surgical light may be classified into a large light spot range and a small light spot range, or a large light spot range, a medium light spot range, and a small light spot range. The second light spot value and the third light spot value are in the small light spot range or the medium light spot range.

Because the specific process of adjusting the parameter(s) of the surgical light and the achieved technical effects have been described in the system embodiment, details are not described herein again.

This specification is described with reference to various exemplary embodiments. However, those skilled in the art will recognize that changes and amendments can be made to the exemplary embodiments without departing from the scope of this specification.

In addition, as understood by those skilled in the art, the principles of this specification may be reflected in a computer program product on a computer-readable storage medium which is pre-installed with computer-readable program code. Any tangible, non-transitory computer-readable storage medium can be used, which includes a magnetic storage device (a hard disk, a floppy disk, or the like), an optical storage device (a CD-ROM, a DVD, a Blu Ray disk, or the like), a flash memory, and/or the like. These computer program instructions may be loaded onto a general-purpose computer, a special-purpose computer, or other programmable data processing devices to form a machine, such that these instructions executed on the computer or the other programmable data processing devices can generate an apparatus for implementing specified functions. These computer program instructions may be stored in a computer-readable memory which can instruct a computer or other programmable data processing devices to work in a specific manner, so that the instructions stored in the computer-readable memory can form an artifact comprising an apparatus for implementing specified functions. For example, as shown in Fig. 1, the surgical lighting device may further include a memory 50 storing a computer program, the computer program comprising a program instruction. The processor 20 is configured to invoke the program instruction to perform the above-described method for adjusting surgical light parameters. The computer program instructions may be alternatively loaded onto a computer or other programmable data processing devices, so that a series of operations and steps are performed on the computer or the other programmable devices, thereby generating a computer-implemented process. Therefore, the instructions executed on the computer or the other programmable devices may provide steps for implementing specified functions.

Although the principles of this specification have been shown in the various embodiments, many modifications of structures, arrangements, ratios, elements, materials, and components that are particularly applicable to specific environments and operational requirements can be made without departing from the principles and scope of the present disclosure.

The foregoing specific description has been described with reference to the various embodiments. However, those skilled in the art will recognize that various amendments and changes can be made without departing from the scope of the present disclosure. Therefore, consideration of the present disclosure is in an illustrative rather than a restrictive sense, and all such modifications shall fall within the scope of the present disclosure. Likewise, advantages of the various embodiments, other advantages, and solutions to the problems are described above. However, the benefits, advantages, solutions to the problems, and any element that can produce these, or solutions that make them more explicit should not be interpreted as critical, essential, or necessary. The term "include" and any other variations thereof used in this specification are non-exclusive. Therefore, a process, a method, an article, or a device that includes a list of elements includes not only these elements, but also other elements that are not explicitly listed or do not belong to the process, the method, the system, the article, or the device. Furthermore, the term "coupling" and any other variations thereof used in this specification refer to a physical connection, an electrical connection, a magnetic connection, an optical connection, a communication connection, a functional connection, and/or any other connection.

Those skilled in the art will recognize that many changes can be made to the details of the above embodiments without departing from the basic principles of the present invention. Therefore, the scope of the present invention should be determined only by the claims.

The present invention has been described with reference to specific examples, which are merely for the purpose of facilitating understanding of the present invention and are not intended to limit the present invention. For those skilled in the technical field to which the present invention belongs, several simple deductions, variations, or replacements can also be made according to the idea of the present invention.

## Claims

1. A method for adjusting parameter(s) of a surgical light comprising:
acquiring an operation field (01) image signal or a three-dimensional signal of an operation field;
determining surgery information (02) according to the operation field image signal or the three-dimensional signal of the operation field, the surgery information comprising a surgery type or a surgery stage; and
determining a parameter (03) adjustment strategy for the surgical light according to the surgery information, and adjusting the parameter(s) of the surgical light according to the parameter adjustment strategy; wherein determining surgery information according to the operation field image signal or the three-dimensional signal of the operation field comprises:
obtaining feature information in an operation field image or the three-dimensional signal according to the current operation field image signal or the current three-dimensional signal of the operation field; and
determining, according to the feature information, the surgery information corresponding to the feature information; and wherein
said obtaining feature information in an operation field image or the three-dimensional signal according to the operation field image signal or the three-dimensional signal of the operation field, and determining, according to the feature information, the surgery information corresponding to the feature information comprises:
obtaining depth information of an incision in the operation field image or the three-dimensional signal according to the operation field image signal or the three-dimensional signal of the operation field; **characterised in that** the method further comprises:
calculating a change value of the depth information of the incision; and
determining, according to the change value of the depth information of the incision, a surgery stage corresponding to the change value of the depth information of the incision, the surgery stage comprising an early surgery period, a middle surgery period, or a late surgery period.

2. The method of claim 1, wherein said determining a parameter adjustment strategy for a surgical light according to the surgery information, and adjusting the parameter(s) of the surgical light according to the parameter adjustment strategy comprises:
adjusting an illuminance of the surgical light to first illuminance when a current surgery is in the early surgery period;
adjusting an illuminance of the surgical light to second illuminance when a current surgery is in the middle surgery period and setting the second illuminance to vary according to a change in the depth information of the incision; or,
adjusting an illuminance of the surgical light to third illuminance when a current surgery is in the late surgery period, and setting the third illuminance to vary according to a change in the depth information of the incision,
wherein both the first illuminance and the third illuminance are less than or equal to the second illuminance.

3. The method of claim 1, wherein said obtaining feature information in an operation field image according to the operation field image signal, and determining, according to the feature information, the surgery information corresponding to the feature information comprises:
obtaining color information in the operation field image according to the operation field image signal; and
determining a surgery stage of a current surgery according to the color information, the surgery stage comprising an early surgery period, a middle surgery period, or a late surgery period.

4. The method of claim 3, wherein said determining a surgery stage of a current surgery according to the color information comprises:
determining an area of a blood stain region in the operation field image according to the color information; and
determining an incision size according to the area of the blood stain region, and determining the surgery stage of the current surgery according to the incision size.

5. The method of claim 1, wherein said obtaining feature information in the three-dimensional signal according to the three-dimensional signal of the operation field, and determining, according to the feature information, the surgery information corresponding to the feature information comprises:
establishing a three-dimensional model of the operation field according to the three-dimensional signal of the operation field; and
determining an incision size according to the three-dimensional model, and determining a surgery stage of a current surgery according to the incision size.

6. The method of claim 4, wherein said determining a parameter adjustment strategy for a surgical light according to the surgery information, and adjusting the parameter(s) of the surgical light according to the parameter adjustment strategy comprises:
adjusting a light spot size of the surgical light to a first light spot value when the current surgery is in the early surgery period;
adjusting a light spot size of the surgical light to a second light spot value when the current surgery is in the middle surgery period, and setting the second light spot value to vary according to a change in the incision size; or,
adjusting a light spot size of the surgical light to a third light spot value when the current surgery is in the late surgery period, and setting the third light spot value to vary depending on a change in the incision size,
wherein both the second light spot value and the third light spot value are less than or equal to the first light spot value.

7. The method of claim 1, wherein said obtaining feature information in an operation field image or the three-dimensional signal according to the operation field image signal or the three-dimensional signal of the operation field, and determining, according to the feature information, the surgery information corresponding to the feature information comprises:
obtaining human body posture information and surgery bed placement position information in the operation field image or the three-dimensional signal according to the operation field image signal or the three-dimensional signal of the operation field; and
determining, according to the human body posture information and the surgery bed placement position information, a surgery type corresponding to the human body posture information and the surgery bed placement position information.

8. The method of claim 1, wherein said obtaining feature information in an operation field image according to the operation field image signal, and determining, according to the feature information, the surgery information corresponding to the feature information comprises:
obtaining brightness information and human body information in the operation field image according to the operation field image signal, the human body information comprising human body structure information or dynamic organ feature information; and
determining, according to the brightness information and the human body information, a surgery type corresponding to the brightness information and the human body information.

9. The method of claim 1, further comprising:
storing, in a clinical library, parameter adjustment strategies for the surgical lights that correspond to a plurality of surgery information; and
recording actual parameter(s) of the surgical light in a current surgery type or a current surgery stage, and dynamically adjusting, through deep learning, the parameter adjustment strategies for the surgical lights that are stored in the clinical library.

10. A surgical lighting device, comprising:
a sensor assembly (10) configured to generate an operation field image signal or a three-dimensional signal of an operation field;
a memory (50) storing a computer program, the computer program comprising a program instruction;
a processor (20) configured to invoke the program instruction to perform the method of any of claims 1 to 9; and
a surgical light (30) configured to illuminate a surgical site according to the parameter(s).

11. A computer-readable storage medium storing a computer program, the computer program comprising a program instruction that, when executed by a processor, causes the processor to perform the method of any of claims 1 to 9.

## Patentansprüche

1. Verfahren zum Einstellen von Parametern einer Operationsleuchte, das Folgendes umfasst:
Erfassen eines Bildsignals oder eines dreidimensionalen Signals eines Operationsfeldes (01);
Bestimmen von Operationsinformationen (02) entsprechend dem Bildsignal oder dem dreidimensionalen Signal des Operationsfeldes, wobei die Operationsinformationen einen Operationstyp oder eine Operationsphase beinhalten; und
Bestimmen einer Parametereinstellstrategie (03) für die Operationsleuchte entsprechend den Operationsinformationen und Einstellen des/der Parameter(s) der Operationsleuchte entsprechend der Parametereinstellstrategie; wobei die Bestimmung von Operationsinformationen anhand des Bildsignal oder des dreidimensionalen Signals des Operationsfeldes Folgendes umfasst:
Erfassen von Merkmalsinformationen in einem Operationsfeldbild oder dem dreidimensionalen Signal entsprechend dem aktuellen Operationsfeldbildsignal oder dem aktuellen dreidimensionalen Signal des Operationsfeldes; und
Bestimmen der Operationsinformation entsprechend der Merkmalsinformation, die mit der Merkmalsinformation übereinstimmt; und wobei das Erfassen von Merkmalsinformation in einem Operationsfeldbild oder dem dreidimensionalen Signal entsprechend dem Operationsfeldbildsignal oder dem dreidimensionalen Signal des Operationsfeldes und
Bestimmen, entsprechend der Merkmalsinformation, der der Merkmalsinformation entsprechenden Operationsinformation, was Folgendes beinhaltet:
Erfassen von ausführlichen Informationen über einen Schnitt in dem Operationsfeldbild oder dem dreidimensionalen Signal entsprechend dem Operationsfeldbildsignal oder dem dreidimensionalen Signal des Operationsfeldes; **dadurch gekennzeichnet, dass** das Verfahren ferner Folgendes umfasst:
Berechnen eines Änderungswerts der ausführlichen Information des Einschnitts, und
Bestimmen, entsprechend dem Änderungswert der ausführlichen Information des Einschnitts, einer Operationsphase, wobei diese eine frühe, eine mittlere oder eine späte Operationsphase beinhaltet.

2. Verfahren nach Anspruch 1, wobei das Bestimmen einer Parametereinstellstrategie für eine Operationsleuchte gemäß den Operationsinformationen und das Einstellen des/der Parameter(s) der Operationsleuchte gemäß der Parametereinstellstrategie Folgendes beinhaltet:
Einstellen der Beleuchtungsstärke der Operationsleuchte auf eine erste Beleuchtungsstärke, wenn sich eine laufende Operation in der frühen Operationsphase befindet,
Einstellen einer Beleuchtungsstärke der Operationsleuchte auf eine zweite Beleuchtungsstärke, wenn sich eine laufende Operation in der mittleren Operationsphase befindet, und Einstellen der zweiten Beleuchtungsstärke, um entsprechend einer Änderung der ausführlichen Information des Einschnitts zu variieren; oder,
Einstellen einer Beleuchtungsstärke der Operationsleuchte auf eine dritte Beleuchtungsstärke, wenn sich eine laufende Operation in der späten Operationsphase befindet, und Einstellen der dritten Beleuchtungsstärke, um entsprechend einer Änderung der ausführlichen Information des Einschnitts zu variieren, wobei sowohl die erste als auch die dritte Beleuchtungsstärke kleiner oder gleich der zweiten Beleuchtungsstärke sind.

3. Verfahren nach Anspruch 1, wobei das Erfassen von Merkmalsinformationen in einem Operationsfeldbild gemäß dem Operationsfeldbildsignal und das Bestimmen der den Merkmalsinformationen entsprechenden Operationsinformationen Folgendes beinhaltet: Erfassen von Farbinformationen im Operationsfeldbild entsprechend dem Operationsfeldbildsignal, und
Bestimmen einer Operationsphase einer aktuellen Operation entsprechend der Farbinformation, wobei die Operationsphase eine frühe, eine mittlere oder eine späte Operationsphase umfasst.

4. Verfahren nach Anspruch 3, wobei die Bestimmung einer Operationsphase einer aktuellen Operation anhand der Farbinformationen Folgendes beinhaltet:
Bestimmen einer Stelle eines Blutfleckbereichs in dem Bild des Operationsfeldes entsprechend der Farbinformation; und
Bestimmen einer Schnittgröße entsprechend der Fläche des Blutfleckenbereichs und-
Bestimmen der Eingriffsphase der laufenden Operation entsprechend der Schnittgröße.

5. Verfahren nach Anspruch 1, wobei das Erhalten von Merkmalsinformationen in dem dreidimensionalen Signal entsprechend dem dreidimensionalen Signal des Operationsfeldes und das Bestimmen, entsprechend den Merkmalsinformationen, den Merkmalsinformationen entsprechende Operationsinformationen beinhaltet:
Aufbau eines dreidimensionalen Modells des Operationsfeldes entsprechend dem dreidimensionalen Signal des Operationsfeldes; und
Bestimmen einer Schnittgröße gemäß dem dreidimensionalen Modell sowie Bestimmen einer Eingriffsphase einer laufenden Operation gemäß der Schnittgröße.

6. Verfahren nach Anspruch 4, wobei das Bestimmen einer Parametereinstellstrategie für eine Operationsleuchte gemäß den Operationsinformationen und das Einstellen des/der Parameter(s) der Operationsleuchte gemäß der Parametereinstellstrategie Folgendes beinhaltet:
Einstellen einer Lichtpunktgröße der Operationsleuchte auf einen ersten Lichtpunktwert, wenn sich die aktuelle Operation in der frühen Operationsphase befindet,
Einstellen einer Lichtpunktgröße der Operationsleuchte auf einen zweiten Lichtpunktwert, wenn sich die laufende Operation in der mittleren Operationsphase befindet, und Einstellen des zweiten Lichtpunktwerts, um entsprechend einer Änderung der Schnittgröße zu variieren; oder,
Einstellen einer Lichtpunktgröße der Operationsleuchte auf einen dritten Lichtpunktwert, wenn sich die laufende Operation in der späten Operationsphase befindet, und Einstellen des dritten Lichtpunktwertes, um je nach einer Änderung der Schnittgröße zu variieren, wobei sowohl der zweite als auch der dritte Lichtpunktwert kleiner oder gleich dem ersten Lichtpunktwert sind.

7. Verfahren nach Anspruch 1, wobei das Erfassen von Merkmalsinformationen in einem Operationsfeldbild oder dem dreidimensionalen Signal entsprechend dem Operationsfeldbildsignal oder dem dreidimensionalen Signal des Operationsfeldes, und das Bestimmen der den Merkmalsinformationen entsprechenden Operationsinformationen gemäß den Merkmalsinformationen Folgendes beinhaltet:
Erfassen von Informationen über die menschliche Körperhaltung und die Position des Operationstisches in dem Operationsfeldbild oder dem dreidimensionalen Signal entsprechend dem Operationsfeldbildsignal oder dem dreidimensionalen Signal des Operationsfeldes; und
Bestimmen eines Operationstyps, der der Information über die Körperhaltung des Menschen und der Information über die Position des Operationstisches entspricht, anhand der Information über die Körperhaltung des Menschen und der Information über die Position des Operationstisches.

8. Verfahren nach Anspruch 1, wobei das Erfassen von Merkmalsinformationen in einem Operationsfeldbild gemäß dem Operationsfeldbildsignal und das Bestimmen der den Merkmalsinformationen entsprechenden Operationsinformationen gemäß den Merkmalsinformationen Folgendes beinhaltet:
Erfassen von Helligkeitsinformationen und Informationen über den menschlichen Körper in dem Operationsfeldbild entsprechend dem Operationsfeldbildsignal, wobei die Informationen über den menschlichen Körper Informationen über die Struktur des menschlichen Körpers oder Informationen über dynamische Organeigenschaften beinhalten; und
Bestimmen eines Operationstyps, der der Helligkeitsinformation und der Information über den menschlichen Körper entspricht, anhand der Helligkeitsinformation und der Information über den menschlichen Körper.

9. Das Verfahren nach Anspruch 1 beinhaltet ferner:
In einer klinischen Bibliothek werden Parametereinstellstrategien für die Operationsleuchten gespeichert, die einer Vielzahl von Operationsinformationen entsprechen; und
Aufzeichnung des(r) tatsächlichen Parameter(s) der Operationsleuchte in einem aktuellen Operationstyp oder einer aktuellen Operationsphase und dynamische Anpassung durch Deep-Learning der Parameteranpassungsstrategien für die Operationsleuchten, die in der klinischen Bibliothek gespeichert sind.

10. Chirurgische Beleuchtungsvorrichtung mit:
einer Sensoreinheit (10), die so ausgelegt ist, dass sie ein Operationsfeldbildsignal oder ein dreidimensionales Signal eines Operationsfeldes erzeugt;
einem Speicher (50), in dem ein Computerprogramm gespeichert ist, wobei das Computerprogramm einen Programmbefehl beinhaltet;
einem Prozessor (20), der so ausgelegt ist, dass er die Programmanweisung aufruft, um das Verfahren nach einem der Ansprüche 1 bis 9 durchzuführen; und
einer Operationsleuchte (30), die so ausgelegt ist, dass sie eine Operationsstelle entsprechend dem/den Parameter(n) beleuchtet.

11. Computerlesbares Speichermedium, das ein Computerprogramm speichert, wobei dieses eine Programmanweisung beinhaltet, die, wenn sie von einem Prozessor ausgeführt wird, diesen veranlasst, das Verfahren nach einem der Ansprüche 1 bis 9 durchzuführen.

## Revendications

1. Procédé de réglage de paramètre(s) d'un éclairage chirurgical comprenant les étapes consistant à :
acquérir un signal d'image de champ d'opération (01) ou un signal tridimensionnel d'un champ d'opération ;
déterminer des informations de chirurgie (02) en fonction du signal d'image de champ d'opération ou du signal tridimensionnel du champ d'opération, les informations de chirurgie comprenant un type de chirurgie ou un stade de chirurgie ; et
déterminer une stratégie de réglage de paramètre(s) (03) pour l'éclairage chirurgical en fonction des informations de chirurgie, et régler le(s) paramètre(s) de l'éclairage chirurgical en fonction de la stratégie de réglage de paramètre(s) ; où le fait de déterminer des informations de chirurgie en fonction du signal d'image de champ d'opération ou du signal tridimensionnel du champ d'opération comprend :
le fait d'obtenir des informations de caractéristique dans une image de champ d'opération ou le signal tridimensionnel en fonction du signal actuel d'image de champ d'opération ou du signal tridimensionnel actuel du champ d'opération ; et
le fait de déterminer, en fonction des informations de caractéristique, les informations de chirurgie correspondant aux informations de caractéristique ; et où ledit fait d'obtenir des informations de caractéristique dans une image de champ d'opération ou le signal tridimensionnel en fonction du signal d'image de champ d'opération ou du signal tridimensionnel du champ d'opération, et de déterminer, en fonction des informations de caractéristique, les informations de chirurgie correspondant aux informations de caractéristique comprend :
le fait d'obtenir des informations de profondeur d'une incision dans l'image de champ d'opération ou le signal tridimensionnel en fonction du signal d'image de champ d'opération ou du signal tridimensionnel du champ d'opération ; **caractérisé en ce que** le procédé comprend en outre :
le fait de calculer une valeur d'évolution des informations de profondeur de l'incision ; et
le fait de déterminer, en fonction de la valeur d'évolution des informations de profondeur de l'incision, un stade de chirurgie correspondant à la valeur d'évolution des informations de profondeur de l'incision, le stade de chirurgie comprenant une période de chirurgie initiale, une période de chirurgie intermédiaire, ou une période de chirurgie finale.

2. Procédé selon la revendication 1, dans lequel ledit fait de déterminer une stratégie de réglage de paramètre(s) pour un éclairage chirurgical en fonction des informations de chirurgie, et de régler le(s) paramètre(s) de l'éclairage chirurgical en fonction de la stratégie de réglage de paramètre(s) comprend :
le fait de régler un éclairement lumineux de l'éclairage chirurgical à un premier éclairement lumineux lorsqu'une stratégie actuelle se trouve dans la période de chirurgie initiale ;
le fait de régler un éclairement lumineux de l'éclairage chirurgical à un deuxième éclairement lumineux lorsqu'une stratégie actuelle se trouve dans la période de chirurgie intermédiaire et de paramétrer le deuxième éclairement lumineux pour varier en fonction d'une évolution des informations de profondeur de l'incision ; ou,
le fait de régler un éclairement lumineux de l'éclairage chirurgical à un troisième éclairement lumineux lorsqu'une stratégie actuelle se trouve dans la période de chirurgie finale, et de paramétrer le troisième éclairement lumineux pour varier en fonction d'une évolution des informations de profondeur de l'incision,
où le premier éclairement lumineux et le troisième éclairement lumineux sont tous deux inférieurs ou égaux au deuxième éclairement lumineux.

3. Procédé selon la revendication 1, dans lequel ledit fait d'obtenir des informations de caractéristique dans une image de champ d'opération en fonction du signal d'image de champ d'opération, et de déterminer, en fonction des informations de caractéristique, les informations de chirurgie correspondant aux informations de caractéristique comprend :
le fait d'obtenir des informations de couleur dans l'image de champ d'opération en fonction du signal d'image de champ d'opération ; et
le fait de déterminer un stade de chirurgie d'une chirurgie actuelle en fonction des informations de couleur, le stade de chirurgie comprenant une période de chirurgie initiale, une période de chirurgie intermédiaire, ou une période de chirurgie finale.

4. Procédé selon la revendication 3, dans lequel ledit fait de déterminer un stade de chirurgie d'une chirurgie actuelle en fonction des informations de couleur comprend :
le fait de déterminer une surface d'une région de couleur sang dans l'image de champ d'opération en fonction des informations de couleur ; et
le fait de déterminer une taille d'incision en fonction de la surface de la région de couleur sang, et de déterminer le stade de chirurgie de la chirurgie actuelle en fonction de la taille d'incision.

5. Procédé selon la revendication 1, dans lequel ledit fait d'obtenir des informations de caractéristique dans le signal tridimensionnel en fonction du signal tridimensionnel du champ d'opération, et de déterminer, en fonction des informations de caractéristique, les informations de chirurgie correspondant aux informations de caractéristique comprend :
le fait d'établir un modèle tridimensionnel du champ d'opération en fonction du signal tridimensionnel du champ d'opération ; et
le fait de déterminer une taille d'incision en fonction du modèle tridimensionnel, et de déterminer un stade de chirurgie d'une chirurgie actuelle en fonction de la taille d'incision.

6. Procédé selon la revendication 4, dans lequel ledit fait de déterminer une stratégie de réglage de paramètre(s) pour un éclairage chirurgical en fonction des informations de chirurgie, et de régler le(s) paramètre(s) de l'éclairage chirurgical en fonction de la stratégie de réglage de paramètre(s) comprend :
le fait de régler une taille de point lumineux de l'éclairage chirurgical à une première valeur de point lumineux lorsque la chirurgie actuelle se trouve dans la période de chirurgie initiale ;
le fait de régler une taille de point lumineux de l'éclairage chirurgical à une deuxième valeur de point lumineux lorsque la chirurgie actuelle se trouve dans la période de chirurgie intermédiaire, et de paramétrer la deuxième valeur de point lumineux pour varier en fonction d'une évolution de la taille d'incision ; ou,
le fait de régler une taille de point lumineux de l'éclairage chirurgical à une troisième valeur de point lumineux lorsque la chirurgie actuelle se trouve dans la période de chirurgie finale, et de paramétrer la troisième valeur de point lumineux pour varier en fonction d'une évolution de la taille d'incision,
où la deuxième valeur de point lumineux et la troisième valeur de point lumineux sont toutes deux inférieures ou égales à la première valeur de point lumineux.

7. Procédé selon la revendication 1, dans lequel ledit fait d'obtenir des informations de caractéristique dans une image de champ d'opération ou le signal tridimensionnel en fonction du signal d'image de champ d'opération ou du signal tridimensionnel du champ d'opération, et de déterminer, en fonction des informations de caractéristique, les informations de chirurgie correspondant aux informations de caractéristique comprend :
le fait d'obtenir des informations de posture de corps humain et des informations de position de placement de lit de chirurgie dans l'image de champ d'opération ou le signal tridimensionnel en fonction du signal d'image de champ d'opération ou du signal tridimensionnel du champ d'opération ; et
le fait de déterminer, en fonction des informations de posture de corps humain et des informations de position de placement de lit de chirurgie, un type de chirurgie correspondant aux informations de posture de corps humain et aux informations de position de placement de lit de chirurgie.

8. Procédé selon la revendication 1, dans lequel ledit fait d'obtenir des informations de caractéristique dans une image de champ d'opération en fonction du signal d'image de champ d'opération, et de déterminer, en fonction des informations de caractéristique, les informations de chirurgie correspondant aux informations de caractéristique comprend :
le fait d'obtenir des informations de luminosité et des informations de corps humain dans l'image de champ d'opération en fonction du signal d'image de champ d'opération, les informations de corps humain comprenant des informations de structure de corps humain ou des informations de caractéristique d'organe dynamique ; et
le fait de déterminer, en fonction des informations de luminosité et des informations de corps humain, une type de chirurgie correspondant aux informations de luminosité et aux informations de corps humain.

9. Procédé selon la revendication 1, comprenant en outre :
le fait de stocker, dans un bibliothèque clinique, des stratégies de réglage de paramètre(s) pour les éclairages chirurgicaux qui correspondent à une pluralité d'informations de chirurgie ; et
le fait d'enregistrer un (des) paramètre(s) actuel(s) de l'éclairage chirurgical dans un type de chirurgie actuel ou un stade de chirurgie actuel, et de régler de manière dynamique, grâce à un apprentissage profond, les stratégies de réglage de paramètre(s) pour les éclairages chirurgicaux qui sont stockées dans la bibliothèque clinique.

10. Dispositif d'éclairage chirurgical, comprenant :
un ensemble de capteur (10) configuré pour générer un signal d'image de champ d'opération ou un signal tridimensionnel d'un champ d'opération ;
une mémoire (50) stockant un programme informatique, le programme informatique comprenant une instruction de programme ;
un processeur (20) configuré pour appeler l'instruction de programme afin de réaliser le procédé selon l'une quelconque des revendications 1 à 9 ; et
un éclairage chirurgical (30) configuré pour éclairer un site chirurgical en fonction du (des) paramètre(s).

11. Support de stockage lisible par ordinateur stockant un programme informatique, le programme informatique comprenant une instruction de programme qui, lorsqu'elle est exécutée par le processeur, amène le processeur à réaliser le procédé selon l'une quelconque des revendications 1 à 9.
